(19) Europäisches Patentamt
European Patent Office
Office européen des brevets

(11) Veröffentlichungsnummer : **0 008 447 B1**

(12) **EUROPÄISCHE PATENTSCHRIFT**

(45) Veröffentlichungstag der Patentschrift : **21.10.81**

(51) Int. Cl.³ : **C 07 D213/20,** C 25 D 3/18

(21) Anmeldenummer : **79103013.3**

(22) Anmeldetag : **17.08.79**

# (54) N-(2,3-Dihydroxypropyl)-pyridiniumsulfate, deren Herstellung sowie diese enthaltende saure galvanische Nickelbäder.

(30) Priorität : **21.08.78 DE 2836581**

(43) Veröffentlichungstag der Anmeldung : **05.03.80 (Patentblatt 80/05)**

(45) Bekanntmachung des Hinweises auf die Patenterteilung : **21.10.81 Patentblatt 81/42**

(84) Benannte Vertragsstaaten : **CH FR GB IT SE**

(56) Entgegenhaltungen :
**CH - A - 205 767**
**DE - A - 1 470 061**
**FR - A - 1 570 155**
**US - A - 2 590 126**

(73) Patentinhaber : **Henkel Kommanditgesellschaft auf Aktien**
**-Patentabteilung- Postfach 1100 Henkelstrasse 67**
**D-4000 Düsseldorf 1 (DE)**

(72) Erfinder : **Willmund, Wolf-Dieter, Dr.**
**Einsteinstrasse 9**
**D-4000 Düsseldorf (DE)**
Erfinder : **Wagner, Heinz, Dr.**
**Goeschweg 25**
**D-4010 Hilden (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

EP 0 008 447 B1

« N-(2,3-Dihydroxypropyl)-pyridiniumsulfate, deren Herstellung sowie diese enthaltende saure galvanische Nickelbäder »

Gegenstand der Erfindung sind N-(2,3-Dihydroxypropyl)-pyridiniumsulfate, deren Herstellung sowie diese enthaltente saure galvanische Nickelbäder.

Es wurde gefunden, daß N-(2,3-Dihydroxypropyl)-pyridiniumsulfate der allgemeinen Formel

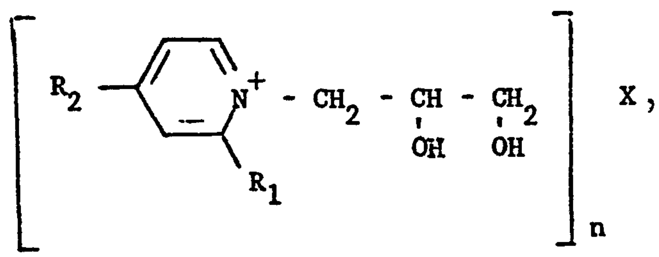

in der $R_1$ und $R_2$ einzeln oder auch gemeinsam Wasserstoff oder einen Methylrest, ferner X den Säurerest $SO^2{}_4^-$bzw. $HSO_4^-$ und n jeweils entsprechend den Wert 2 oder 1 darstellen, neue wertvolle Einebnungsmittel für galvanische Nickelbäder bilden.

Zu den neutralen Sulfaten gelangt man, wenn man Pyridin, beziehungsweise dessen entsprechende Substitutionsprodukte zunächst in die Sulfate überführt und dann mit Glycid umsetzt. Die sauren Sulfate werden erhalten, wenn man Pyridin bzw. dessen entsprechende Substitutionsprodukte mit Glycerinchlorhydrin quaterniert, das gewonnene quartäre Ammoniumsalz mit Schwefelsäure versetzt und die frei werdende Salzsäure entfernt.

Es ist bereits bekannt, von heterocyclischen Stickstoffbasen wie Pyridin, Chinolin, Isochinolin, Chinaldin abgeleitete quartäre Ammoniumsalze als galvanische Zusatzmittel in sauren Nickelbädern zu verwenden. Die mit Hilfe dieser Zusatzmittel erzeugten Nickelüberzüge sind vielfach spröde oder weisen in den niederen Stromdichtebereichen Mattzonen auf und genügen somit nicht den an sie gestellten Anforderungen.

Weiterhin ist es bereits bekannt, Umsetzungsprodukte von heterocyclischen Stickstoffbasen vom aromatischen Typ mit Sultonen als Einebnungsmittel in galvanischen Nickelbädern zu verwenden. Diese Produkte stellen nach ihrem chemischen Aufbau innere Salze von Aminoalkansulfonsäuren, sogenannte Sulfobetaine, dar. Der Einebnungseffekt dieser Produkte ist durchaus befriedigend. Sie unterliegen aber unter gewissen Bedingungen der Zersetzung, durch die der Einebnungseffekt beeinträchtigt werden kann.

Es stellte sich daher die Aufgabe, Produkte aufzufinden, die gleichfalls befriedigende Einebnungseffekte liefern, ohne vergleichsweise anfällig für eine Zersetzung im sauren galvanischen Nickelbad zu sein wie die Sulfobetaine.

Diese Aufgabe wurde dadurch gelöst, daß man saure galvanische Nickelbäder verwendet, die als Einebnungsmittel N-(2,3-Dihydroxypropyl)-pyridiniumsulfate der Formel

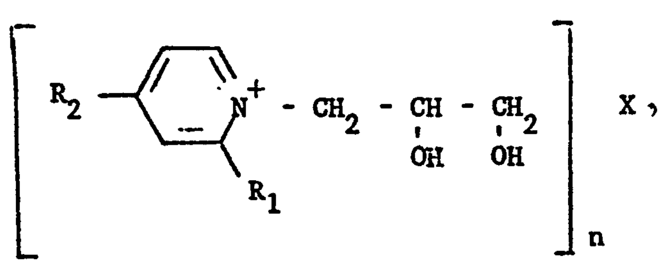

in der $R_1$ und $R_2$ einzeln oder auch gemeinsam Wasserstoff oder einen Methylrest, ferner X den Säurerest $SO^2{}_4^-$ bzw. $HSO^-{}_4$ und n jeweils entsprechend den Wert 2 oder 1 darstellen, enthalten.

Die mit den erfindungsgemäßen Produkten hergestellten Bäder können extrem hohen Badbelastungen ausgesetzt werden, ohne daß störende Zersetzungsprodukte entstehen. Darüber hinaus zeichnen sich die mit den erfindungsgemäßen Produkten hergestellten Bäder durch eine ausgezeichnete Einebnung sowie Einebnungstiefenstreuung aus.

Besondere Bedeutung unter den N-(2,3-Dihydroxypropyl)-pyridiniumsulfaten kommt dabei sowohl von der Zugänglichkeit als auch von der Wirkung den N-(2,3-Dihydroxypropyl)-pyridiniumsulfaten und insbesondere dem sauren Sulfat zu.

Die Anwendungskonzentrationen für die erfindungsgemäßen neuen Verbindungen liegen zwischen 0,05 und 3 g pro Liter, vorzugsweise zwischen 0,1 und 0,5 g pro Liter Badflüssigkeit. Die anwendbaren Stromdichten liegen im Bereich von 0,1 bis 9 A/dm² bei einer Betriebstemperatur von etwa 50 bis 60 °C.

Neben den erfindungsgemäßen Einebnungsmitteln werden den sauren galvanischen Nickelbädern

übliche Grundglanzmittel wie Benzol-m-disulfonsäure, Diaryldisulfimide, Sulfonamide, Saccharin und dergleichen zugesetzt.

Aus der DE-A-1 470 061 war es bereits bekannt, 1,2-Dichlorpropenpyridiniumsalze als primäre Glanzmittel in Vernickelungsbädern einzusetzen. Zur Erzeugung spiegelglänzender Abscheidungen finden diese primären Glanzmittel in Kombination mit sekundären Glanzmitteln sowie sekundären Hilfsglanzmitteln Verwendung.

Im Vergleich zum Erfindungsgegenstand handelt es sich hierbei um eine andere Art von Pyridiniumsalzen, die im Hinblick auf galvanische Nickelbäder einerseits keine Einebnungsmittel darstellen und andererseits nur in der beschriebenen Kombination den erwünschten Erfolg bedingen. Demgegenüber zeichnen sich die erfindungsgemäßen Verbindungen durch eine ausgezeichnete Einebnungswirkung auf, die auch bei hohen Badbelastungen und selbst im Bereich niederer Stromdichten erzielt wird. Mithin wird die erfindungsgemäße Lehre durch den bekannten Stand der Technik in keiner Weise nahegelegt.

## Beispiele

Zunächst wird die Herstellung der neuen Verbindungen beschrieben.

N-(2,3-Dihydroxypropyl)-pyridiniumsulfat

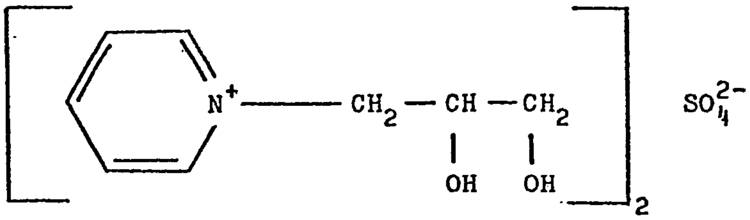

79 g Pyridin (1 Mol) wurden vorgelegt und unter Kühlen bei ca. 20 °C mit einem Gemisch aus 49 g konzentrierter Schwefelsäure und 130 g Wasser (0,5 Mol) versetzt. Nach Erwärmen auf 40 °C wurden 74 g Glycid (1 Mol) langsam innerhalb von 45 Minuten zugetropft. Dabei stieg die Temperatur auf 50 °C. Danach wurde noch 5 Stunden bei etwa 70 °C nachgerührt und anschließend in Vakuum eingeengt. Es wurden 180 g dickflüssiges, schwach gelblichgrün gefärbtes Reaktionsprodukt erhalten. Das Produkt besitzt folgenden Brechungsindex: $n_D^{20}$ 1,5140.

Für den Einsatz in galvanischen Nickelbädern wurde eine 20 %ige Lösung hergestellt.

Zur Aufbereitung des Reaktionsproduktes kann man dieses auch mit Wasserdampf behandeln. Nach dem Blasen mit Wasserdampf bestimmt man in einem aliquoten Teil die Aktivsubstanzmenge und stellt danach die Lösung ebenfalls auf 20 Gewichtsprozent Festkörper ein.

In analoger Arbeitsweise zu vorgenannter Herstellungsbeschreibung wurde aus α-Picolin als Ausgangsmaterial die Verbindung N-(2,3-Dihydroxy-propyl)-α-picoliniumsulfat

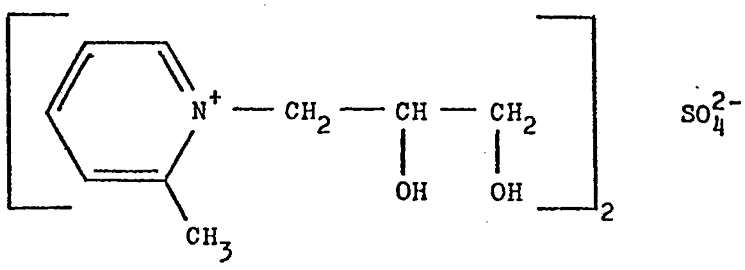

hergestellt, Brechungsindex $n_D^{20}$ 1,5268.

Aus γ-Picolin wurde entsprechend N-(2,3-Dihydroxypropyl)-γ-picoliniumsulfat

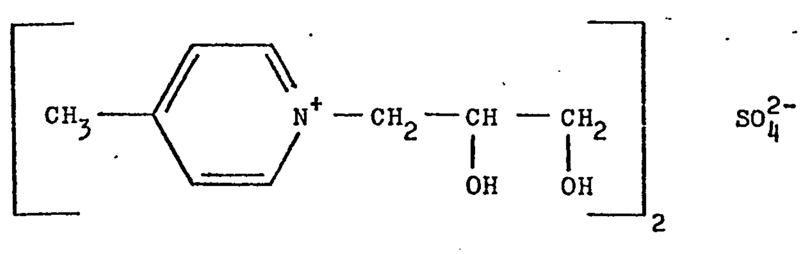

erhalten, Brechungsindex $n_D^{20}$ 1,5272.

Der Einsatz von α,γ-Lutidin als Ausgangsmaterial führt zu der Verbindung N-(2,3-Dihydroxypropyl)-α,γ-lutidiniumsulfat

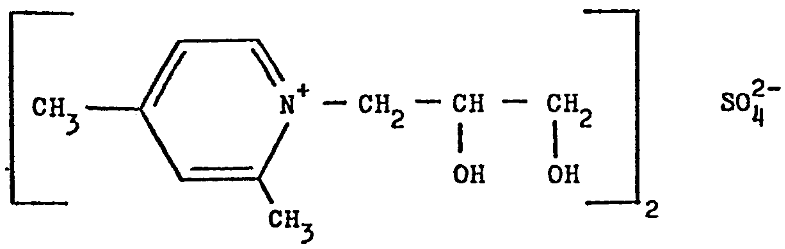

N-(2,3-Dihydroxypropyl)-pyridiniumhydrogensulfat

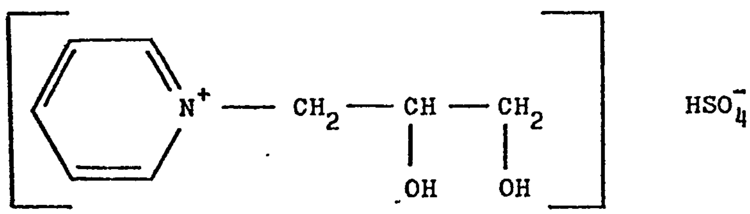

79 g Pyridin (1 Mol) wurden vorgelegt und bei 100 °C unter Rühren mit 110,5 g (1 Mol) α-Glycerinchlorhydrin versetzt. Es wurde anschließend 5 Stunden bei 100-110 °C nachgerührt, das Produkt nach dem Abkühlen in Wasser aufgenommen und Wasserdampt hindurchgeblasen, um etwa nicht umgesetzte Pyridinreste zu entfernen. Hernach wurde mit der molaren Menge Schwefelsäure versetzt. Durch Einengen im Vakuum wurde die frei gewordene Salzsäure entfernt. Für den Einsatz in galvanischen Bädern wurde eine 20 %ige Lösung hergestellt.

Die reine Substanz wurde durch Lösen des eingeengten Rohproduktes in heißem Äthanol und wenig Wasser nach Abkühlen und Stehen über Nacht kristallin erhalten. Sie stellt ein fast weißes, feinkristallines, lockeres Pulver dar, das bei 184-186 °C schmilzt.

In analoger Weise lassen sich das N-(2,3-Dihydroxypropyl)-α-picolinium-, N-(2,3-Dihydroxypropyl) γ-picolinium- und N-(2,3-Dihydroxypropyl)-α,γ-lutidinium-hydrogensulfat erhalten.

Nachfolgend werden Beispiele für mit den erfindungsgemäßen Verbindungen hergestellte Nickelbäder angegeben.

Beispiel 1

Versetzt man ein Nickelbad vom Watts-Typ mit 2 g/l o-Toluoldisulfonimid als Grundglanzmittel, 0,5 g/l Natriumdodecylsulfat als Netzmittel und 0,25 g/l N-(2,3-Dihydroxypropyl)-pyridiniumsulfat als Einebnungsmittel, so werden bei einer Badtemperatur von 50 bis 60 °C im Stromdichtebereich von 0,5 bis 9 A/dm$^2$ hochglänzende, stark eingeebnete Nickelniederschläge von guter Duktilität erhalten. Nach Badbelastungen von 1 000 Ah/l sind störende Zersetzungsprodukte in den Bädern nicht nachweisbar.

An die Stelle des N-(2,3-Dihydroxypropyl)-pyridiniumsulfats können mit gleich gutem Erfolg in gleicher Konzentration die Produkte N-(2,3-Dihydroxypropyl)-α-picoliniumsulfat, N-(2,3-Dihydroxypropyl)-γ-picoliniumsulfat und N-(2,3-Dihydroxypropyl)-α,γ-lutidiniumsulfat und mit besonderem Vorteil das N-(2,3-Dihydroxypropyl)-pyridiniumhydrogensulfat treten.

Beispiel 2

Versetzt man ein Nickelbad vom Watts-Typ mit 1,5 g/l N-(Benzolsulfonyl)-acetamid als Grundglanzmittel, 0,5 g/l Natriumdodecylsulfat als Netzmittel und 0,3 g/l N-(2,3-Dihydroxypropyl)-pyridiniumsulfat als Einebnungsmittel, so werden bei einer Badtemperatur von 50 bis 60 °C im Stromdichtebereich von 0,1 bis 9 A/dm$^2$ hochglänzende, guteingeebnete Nickelniederschläge von guter Duktilität erhalten. Der Ersatz durch das entsprechende saure Sulfat liefert gleichfalls hochwertige Nickelniederschläge.

Die nachfolgend aufgeführten Nickelbäder führten zu vergleichsweise gleich guten Ergebnissen.

Beispiel 3

| | | |
|---|---|---|
| Nickelsulfat ($NiSO_4 \cdot 7H_2O$) | 270 | g/l |
| Nickelchlorid ($NiCl_2 \cdot 6H_2O$) | 65 | g/l |
| Borsäure | 35 | g/l |
| Natriumdodecylsulfat | 0,5 | g/l |
| Saccharin | 4,0 | g/l |
| N-(2,3-Dihydroxypropyl)-pyridiniumsulfat | 0,5 | g/l |

Beispiel 4

| | |
|---|---|
| Nickelsulfat ($NiSO_4 \cdot 7H_2O$) | 140 g/l |
| Nickelchlorid ($NiCl_2 \cdot 6H_2O$) | 110 g/l |
| Borsäure | 40 g/l |
| Saccharin | 6 g/l |
| Isononylsulfat, Na-salz | 0,3 g/l |
| N-(2,3-Dihydroxypropyl)-α-picoliniumsulfat | 0,2 g/l |

Beispiel 5

| | |
|---|---|
| Nickelsulfat ($NiSO_4 \cdot 6H_2O$) | 280 g/l |
| Nickelchlorid ($NiCl_2 \cdot 6H_2O$) | 65 g/l |
| Borsäure | 35 g/l |
| Natriumdodecylsulfat | 0,3 g/l |
| Saccharin | 6 g/l |
| Propargylalkohol | 0,013 g/l |
| Allylsulfonat | 1,0 g/l |
| N-(2,3-Dihydroxypropyl)-γ-picoliniumsulfat | 0,3 g/l |

Ein weiterer Vorteil der erfindungsgemäßen Verbindungen liegt in ihrer einfachen Herstellung, zu der kein Propansulton benötigt wird.

**Ansprüche**

1. N-(2,3-Dihydroxypropyl)-pyridiniumsulfate der allgemeinen Formel

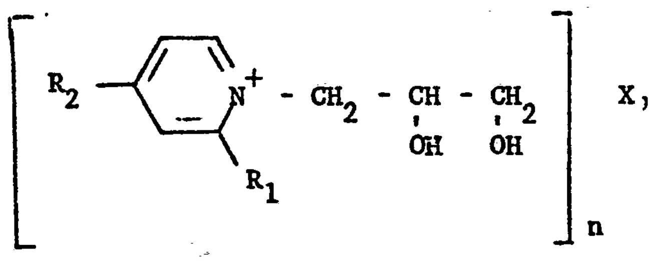

in der $R_1$ und $R_2$ einzeln oder auch gemeinsam Wasserstoff oder einen Methylrest, ferner X den Säurerest $SO_4^{2-}$ bzw. $HSO_4^-$ und n jeweils entsprechend den Wert 2 oder 1 darstellen

2. N-(2,3-Dihydroxypropyl)-pyridiniumsulfat gemäß Anspruch 1.
3. N-(2,3-Dihydroxypropyl)-α-picoliniumsulfat gemäß Anspruch 1.
4. N-(2,3-Dihydroxypropyl)-γ-picoliniumsulfat gemäß Anspruch 1.
5. N-(2,3-Dihydroxypropyl)-α,γ-lutidiniumsulfat gemäß Anspruch 1.
6. N-(2,3-Dihydroxypropyl)-pyridiniumhydrogensulfat gemäß Anspruch 1.
7. Verfahren zur Herstellung der neutralen N-(2,3-Dihydroxypropyl)-pyridiniumsulfate nach Anspruch 1-5, dadurch gekennzeichnet, daß man Pyridin bzw. dessen entsprechende Substitutionsprodukte zunächst in die Sulfate überführt und anschließend mit Glycid umsetzt.
8. Verfahren zur Herstellung der sauren N(2,3-Dihydroxypropyl)-pyridiniumsulfate nach Anspruch 1 und 6, dadurch gekennzeichnet, daß man Pyridin bzw. dessen entsprechende Substitutionsprodukte zunächst mit Glycerinchlorhydrin quaterniert, das gewonnene quartäre Ammoniumsalz mit Schwefelsäure versetzt und die freiwerdende Salzsäure entfernt.
9. Saure galvanische Nickelbäder üblicher Zusammensetzung mit einem Gehalt an Glanz- und Netzmitteln, dadurch gekennzeichnet, daß sie als Einebnungsmittel N-(2,3-Dihydroxypropyl)-pyridiniumsulfate der allgemeinen Formel

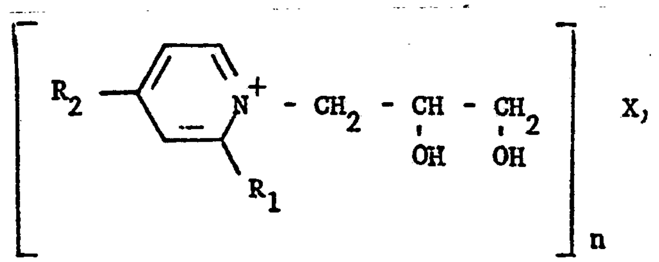

in der $R_1$ und $R_2$ einzeln oder gemeinsam Wasserstoff oder einen Methylrest, ferner X den Säurerest $SO_4^{2-}$ bzw. $HSO_4^-$ und n jeweils entsprechend den Wert 2 oder 1 darstellen, enthalten.

10. Saure galvanische Nickelbäder nach Anspruch 9, dadurch gekennzeichnet, daß sie die N-(2,3-Dihydroxypropyl)-pyridiniumsulfate in einer Menge von 0,05 bis 3 g/l, vorzugsweise 0,1-0,5 g/l Badflüssigkeit enthalten.

11. Saure galvanische Nickelbäder nach Anspruch 9 und 10, die N-(2,3-Dihydroxypropyl)-pyridiniumsulfat enthalten.

12. Saure galvanische Nickelbäder nach Anspruch 9 und 10, die N-(2,3-Dihydroxypropyl)-pyridiniumhydrogensulfat enthalten.

## Claims

1. N-(2,3-dihydroxypropyl)-pyridinium sulfates corresponding to the following general formula

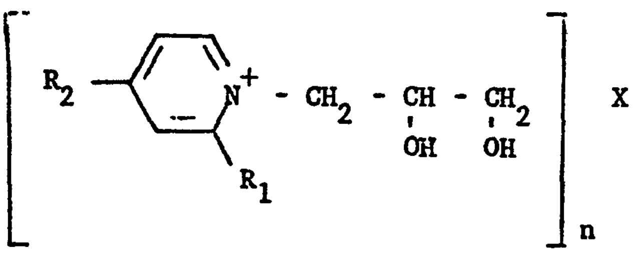

in which $R_1$ and $R_2$ individually or even together represent hydrogen or a methyl radical, X represents the acid residue $SO_4^{2-}$ or $HSO_4^-$ and n correspondingly represents the number 2 or 1.

2. N-(2,3-dihydroxypropyl)-pyridinium sulfate as claimed in Claim 1.

3. N-(2,3-dihydroxypropyl)-α-picolinium sulfate as claimed in Claim 1.

4. N-(2,3-dihydroxypropyl)-γ-picolinium sulfate as claimed in Claim 1.

5. N-(2,3-dihydroxypropyl)-α-γ-lutidinium sulfate as claimed in Claim 1.

6. N-(2,3-dihydroxypropyl)-piridinium hydrogen sulfate as claimed in Claim 1.

7. A process for producing the neutral N-(2,3-dihydroxypropyl)-pyridinium sulfates claimed in Claims 1 to 5, characterised in that pyridine or its corresponding substitution products is first converted into the sulfates and then reacted with glycide.

8. A process for producing the acid N-(2,3-dihydroxypropyl)-pyridinium sulfates claimed in Claims 1 to 6, characterised in that pyridine or its corresponding substitution products is first quaternised with glycerol chlorohydrin, sulfuric acid is added to the quaternary ammonium salt obtained and the hydrochloric acid released is removed.

9. Acid electro-nickel-plating baths of standard composition and containing brightening and wetting agents, characterised in that they contain as levelling agents N-(2,3-dihydroxypropyl)-pyridinium sulfates corresponding to the following general formula

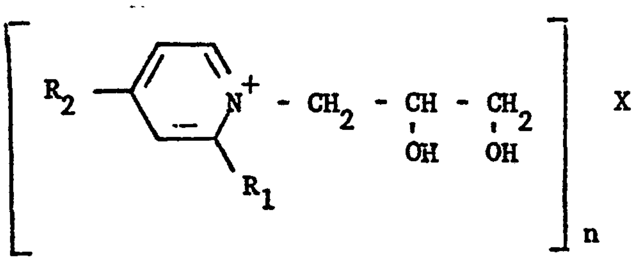

in which $R_1$ and $R_2$ individually or together represent hydrogen or a methyl radical, X represents the acid residue $SO_4^{2-}$ or $HSO_4^-$ and n correspondingly represents the number 1 or 2.

10. Acid electro-nickel-plating baths as claimed in Claim 9, characterised in that they contain N-(2,3-dihydroxypropyl)-pyridinium sulfates in a quantity of from 0.05 to 3 g/l and preferably in a quantity of from 0.1 to 0.5 g/l of bath liquid.

11. Acid electro-nickel-plating baths as claimed in Claims 9 and 10 containing N-(2,3-dihydroxypropyl)-pyridinium sulfate.

12. Acid electro-nickel-plating baths as claimed in Claims 9 and 10 containing N-(2,3-dihydroxypropyl)-pyridinium hydrogen sulfate.

**Revendications**

1. Sulfates de N-(2,3-dihydroxypropyl)-pyridinium de formule générale

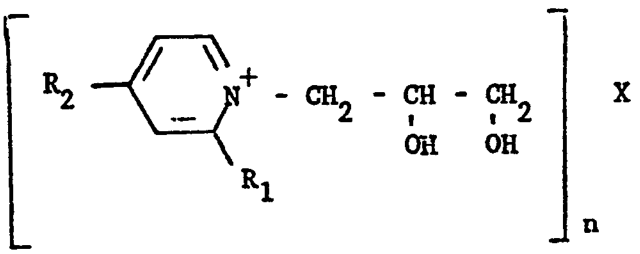

dans laquelle $R_1$ et $R_2$ représentent isolément ou en commun l'hydrogène ou un reste méthyle, X représente le reste acide $SO_4^{-2}$ ou $HSO_4^-$ et n est égal, en correspondance, à 2 ou 1.

2. Sulfate de N-(2,3-dihydroxypropyl)-pyridinium selon la revendication 1.

3. Sulfate de N-(2,3-dihydroxypropyl)-α-picolinium selon la revendication 1.

4. Sulfate de N-(2,3-dihydroxypropyl)-γ-picolinium selon la revendication 1.

5. Sulfate de N-(2,3-dihydroxypropyl)-α,γ-lutidinium selon la revendication 1.

6. Bisulfate de N-(2,3-dihydroxypropyl)-pyridinium selon la revendication 1.

7. Procédé de préparation des sulfates neutres de N-(2,3-dihydroxypropyl)-pyridinium selon les revendications 1 à 5, caractérisé en ce que l'on convertit d'abord la pyridine ou ses produits de substitution correspondants en les sulfates et on fait ensuite réagir avec le glycide.

8. Procédé de préparation des sulfates acides N-(2,3-dihydroxypropyl)-pyridinium selon les revendications 1 et 6, caractérisé en ce que l'on quaternise d'abord la pyridine ou ses produits de substitution correspondants par la chlorhydrine du glycérol, on ajoute au sel d'ammonium quaternaire obtenu de l'acide sulfurique et on élimine l'acide chlorhydrique libéré.

9. Bains galvaniques acides de nickel de composition usuelle contenant des brillanteurs et des agents mouillants, caractérisés en ce qu'ils contiennent en tant qu'agents nivelants des sulfates de N-(2,3-dihydroxypropyl)-pyridinium de formule générale

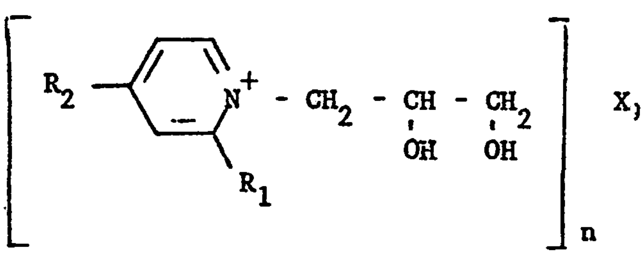

dans laquelle $R_1$ et $R_2$ représentent isolément ou ensemble l'hydrogène ou un reste de méthyle, X représente le reste acide $SO_4^{-2}$ ou $HSO_4^-$ et n, en correspondance, est égal à 2 ou 1.

10. Bains galvaniques acides de nickel selon la revendication 9, caractérisés en ce qu'ils contiennent les sulfates de N-(2,3-dihydroxypropyl)-pyridinium en quantité de 0,05 à 3 g/l, de préférence de 0,1 à 0,5 g/l de liquide du bain.

11. Bains galvaniques acides de nickel selon les revendications 9 et 10, caractérisés en ce qu'ils contiennent du sulfate de N-(2,3-dihydroxypropyl)-pyridinium.

12. Bains galvaniques acides de nickel selon les revendications 9 et 10, caractérisés en ce qu'ils contiennent du bisulfate de N-(2,3-dihydroxypropyl)-pyridinium.